(19) Europäisches Patentamt
European Patent Office
Office européen des brevets

(11) EP 0 603 132 B1

(12) **EUROPÄISCHE PATENTSCHRIFT**

(45) Veröffentlichungstag und Bekanntmachung des Hinweises auf die Patenterteilung:
**12.05.1999 Patentblatt 1999/19**

(51) Int Cl.[6]: **C07D 487/04**, C09B 67/00, C09B 67/18, C09B 57/00 // (C07D487/04, 209:00, 209:00)

(21) Anmeldenummer: **93810863.6**

(22) Anmeldetag: **09.12.1993**

(54) **Mischkristalle von sulfonierten Diketopyrrolopyrrolen**

Mixed crystals of sulfonated diketopyrrolopyrroles

Cristaux mixtes de dicétopyrrolopyrroles sulfonés

(84) Benannte Vertragsstaaten:
**CH DE FR GB LI**

(30) Priorität: **18.12.1992 CH 3894/92**

(43) Veröffentlichungstag der Anmeldung:
**22.06.1994 Patentblatt 1994/25**

(73) Patentinhaber: **Ciba Specialty Chemicals Holding Inc.**
**4057 Basel (CH)**

(72) Erfinder:
- **Hari, Stefan, Dr.**
  **CH-4153 Reinach (CH)**
- **Wallquist, Olof, Dr.**
  **CH-1723 Marly (CH)**
- **Herren, Fritz, Dr.**
  **CH-3186 Düdingen (CH)**
- **Eichenberger, Thomas, Dr.**
  **CH-4056 Basel (CH)**

(56) Entgegenhaltungen:
**EP-A- 0 224 445** **DE-A- 4 011 927**
**DE-A- 4 037 556**

EP 0 603 132 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft Mischkristalle aus Metall- und Aminsalzen von sulfonierten 1,4-Diketo-3,6-diarylpyrrolopyrrolen und ihre Verwendung zum Färben von hochmolekularem organischem Material.

Metall- und Aminsalze von sulfonierten 1,4-Diketo-3,6-diarylpyrrolopyrrolen sind in US-Patent 4 791 204 als Zusatz zu unsulfonierten 1,4-Diketopyrrolopyrrolpigmenten zur Verbesserung gewisser Eigenschaften, wie insbesondere das rheologische Verhalten, Hitzebeständigkeit und Verzugseigenschaften, beschrieben. Sulfonierte Mischungen von mindestens 3 verschiedenen 1,4-Diketo-3,6-diphenylpyrrolopyrrolen können, wie in GB-Patent 2 238 550 beschrieben, zum gleichen Zweck mit zum Teil noch besseren Resultaten ebenfalls als Zusatz zu unsulfonierten 1,4-Diketopyrrolopyrrolpigmenten verwendet werden.

Es ist nun gefunden worden, dass durch Behandlung von Metallsalzen von sulfonierten 1,4-Diketo-3,6-diarylpyrrolopyrrolen mit Aminen überraschenderweise Mischkristallverbindungen entstehen, die sich durch unerwartet hohe Pigmenteigenschaften auszeichnen.

Die vorliegende Erfindung betrifft demnach Mischkristalle aus mindestens zwei voneinander verschiedenen Verbindungen der Formel

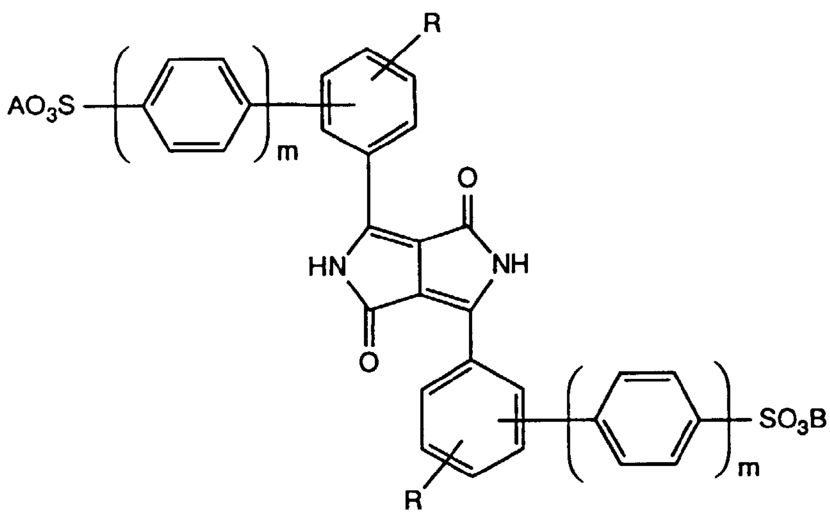

(I),

worin A und B unabhängig voneinander ein Kation der Formeln

$$\frac{M^{+n}}{n} \text{ oder } N^{+}H(R_1)(R_2)(R_3)$$

darstellen, M ein ein-, zwei- oder dreiwertiges Metallkation, n die Zahlen 1, 2 oder 3, $R_1$, $R_2$ und $R_3$ unabhängig voneinander Wasserstoff, $C_1$-$C_{22}$-Alkyl, $C_7$-$C_{24}$-Aralkyl, $C_5$-$C_6$-Cycloalkyl oder $C_6$-$C_{18}$-Aryl bedeuten, m Null oder 1 ist und R Wasserstoff, $C_1$-$C_4$-Alkyl oder $C_1$-$C_4$-Alkoxy bedeutet, dadurch gekennzeichnet, dass in den die Mischkristalle bildenden Verbindungen der Formel I die Kationen A und B eine Zusammensetzung

$$\left(\frac{M^{+n}}{n}\right)_{1-x}\left[N^{+}H(R_1)(R_2)(R_3)\right]_{x}$$

aufweisen, worin x eine Zahl zwischen 0,2 und 0,8, bevorzugt zwischen 0,35 und 0,65 aber insbesondere zwischen 0,4 und 0,6, bedeutet und dass sich das Röntgenbeugungsdiagramm der Mischkristalle vom Röntgenbeugungsdiagramm der Gemische der entsprechenden Verbindungen in welchen x 1 beziehungsweise Null bedeutet, unterscheidet.

Bedeutet m die Zahl 1, so handelt es sich um Verbindungen der Formel

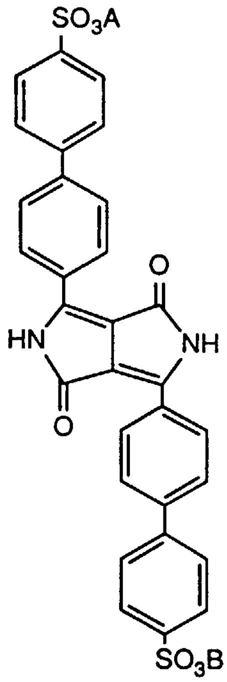

(II)

Stellen A oder B ein Kation der Formel $\frac{M^{+n}}{n}$ dar, so handelt es sich dabei beispielsweise um ein Alkali-, ein Erdalkali- oder ein Uebergangsmetallkation, insbesondere aber um $Na^+$, $K^+$, $Mg^{2+}$, $Ca^{2+}$, $Sr^{2+}$, $Ba^{2+}$, $Mn^{2+}$, $Zn^{2+}$, $Cu^{2+}$, $Ni^{2+}$, $Cd^{2+}$, $Fe^{2+}$, $Fe^{3+}$, $Co^{3+}$, $Al^{3+}$ und $Cr^{3+}$. Bevorzugt werden $Na^+$, $Ca^{2+}$, $Mg^{2+}$, $Sr^{2+}$, $Zn^{2+}$ und $Al^{3+}$.

Bedeuten $R_1$, $R_2$ und $R_3$ $C_1$-$C_{22}$-Alkyl, so handelt es sich dabei z.B. um Methyl, Ethyl, Propyl, Isopropyl, n-Butyl, sek.-Butyl, tert.-Butyl, tert.-Amyl, Octyl, Decyl, Dodecyl, Hexadecyl, Stearyl, Eicosyl oder Docosyl.

Als $C_7$-$C_{24}$-Aralkyl bedeuten $R_1$, $R_2$ und $R_3$ vorzugsweise solche Aralkylgruppen, die eine 1-12, vorzugsweise 1-6 und insbesondere 1-4 C-Atome enthaltende, verzweigte oder unverzweigte Alkylkette (z.B. wie oben ausgeführt) und einen vorzugsweise mono- oder bicyclischen Arylrest enthalten. Als Beispiele seien Benzyl und Phenylethyl genannt.

Sind $R_1$, $R_2$ und $R_3$ Cycloalkyl, so handelt es sich z.B. um Cyclopentyl oder Cyclohexyl.

Bedeuten $R_1$, $R_2$ und $R_3$ $C_6$-$C_{18}$-Aryl, so handelt es sich dabei beispielsweise um Phenyl oder Naphthyl, insbesondere um unsubstituiertes oder durch Halogen, wie Chlor oder Brom, $C_1$-$C_4$-Alkyl oder $C_1$-$C_4$-Alkoxy substituiertes Phenyl.

Bedeuten etwaige Substituenten $C_1$-$C_6$-Alkoxy, dann handelt es sich z.B. um Methoxy, Ethoxy, Propoxy, n-Butoxy oder tert.-Butoxy.

R als $C_1$-$C_4$-Alkyl bedeutet z.B. Methyl, Ethyl, Propyl, Isopropyl, n-Butyl oder tert.-Butyl. Bevorzugt sind Methyl und tert.-Butyl.

Als Beispiele für $N^+H(R_1)(R_2)(R_3)$ seien genannt:

$N^+H_4$, $N^+H_3CH_3$, $N^+H_2(CH_3)_2$, $N^+H_3C_2H_5$, $N^+H_2(C_2H_5)_2$, $N^+H_3C_3H_7$-iso, $N^+H_3C_8H_{17}$, $N^+H_3C_{12}H_{25}$, $N^+H_3C_{18}H_{37}$, $N^+H_3$-Cyclohexyl, $N+H_2(\text{Cyclohexyl})_2$, $N+H_3(CH_3)(C_6H_5)$, $N^+H_3C_6H_5$, $N^+H_3$-para-Toluidin und $N^+H_3$-Benzyl.

Die Herstellung der Mischkristalle gemäss Anspruch 1 erfolgt beispielsweise durch Umsetzung eines mindestens teilweise wasserlöslichen Metallsalzes der Formel

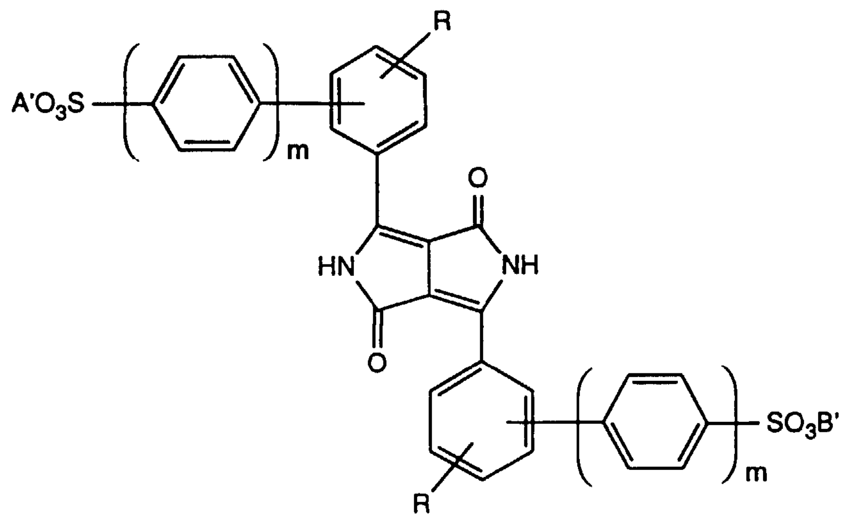

(III),

worin A' und B' unabhängig voneinander ein Kation der Formel $\frac{M^{+n}}{n}$ gemäss der oben angegebenen Definition oder bevorzugt beide $Na^+$ bedeuten, mit einer Verbindung der Formel

$$N^+H(R_1)(R_2)(R_3) \qquad X^- \qquad \text{(IV)},$$

worin $X^-$ ein Anion, der genügende Löslichkeit in Wasser gewährleistet, wie z.B. Sulfat oder insbesondere Chlorid ist, so, dass eine Zusammensetzung

$$\left(\frac{M^{+n}}{n}\right)_{1-x}\left[N^+H(R_1)(R_2)(R_3)\right]_x$$

gemäss der oben angegebenen Definition gewährleistet ist, wobei in den obigen Formeln III und IV m, R, $R_1$, $R_2$ und $R_3$ die oben angegebene Bedeutung haben, nach an sich bekannten Methoden.

Die Umsetzung erfolgt bevorzugt in Wasser, gegebenenfalls mit einem Zusatz an n-Butanol, um die Schaumbildung zu unterdrücken und die Benetzbarkeit der Edukte zu verbessern, bei einer Temperatur zwischen 60 und 120°C während 1 bis 20 Stunden.

Anstatt die Ammoniumverbindung der Formel IV in Form des Chlorids oder Sulfats zuzugeben, kann auch zuerst die äquivalente Menge Säure (z.B. HCl oder $H_2SO_4$), gegebenenfalls in leichtem Ueberschuss, zugegeben werden, und anschliessend das entsprechende freie Amin der Formel $N(R_1)(R_2)(R_3)$ eingetragen werden. Die nachfolgenden Beispiele 1 und 2 betreffen letztere Arbeitsweise.

Vorzugsweise erhält man die erfindungsgemässen Mischkristalle durch Umsetzung eines mindestens teilweise wasserlöslichen Metallsalzes der Formel III, worin A und B $Na^+$ bedeuten, mit einem Gemisch aus Metallsalz der Formel

$$M_1^{+n}(X^-)_n, \qquad \text{(V)},$$

worin $M_1$ eines der obenerwähnten Metallkationen, ausser Na, bedeutet, und Ammoniumsalz der Formel

$$N^+H(R_1)(R_2)(R_3) \qquad X^- \qquad \text{(IV)},$$

wobei $X^-$ ein Anion, der genügende Löslichkeit in Wasser gewährleistet, bevorzugt $Cl^-$, ist.

Anstelle der Ammoniumsalze (z.B. in Form ihrer Chloride) kann wiederum zuerst die äquivalente Menge

Säure, gegebenenfalls in leichtem Ueberschuss, zugegeben werden und anschliessend ein Gemisch von Metallsalz der Formel V und freiem Amin $N(R_1)(R_2)(R_3)$ eingetragen werden. Die nachfolgenden Beispiele 3 bis 6 betreffen letztere Herstellungsmethode.

Bei den Verbindungen der Formeln III und IV und demnach auch V handelt es sich um bekannte Verbindungen.

Von besonderer Bedeutung sind erfindungsgemässe Mischkristalle aus mindestens zwei voneinander verschiedenen Verbindungen der Formel I, worin A und B unabhängig voneinander ein Kation ausgewählt aus $Na^+$, $Ca^{2+}$, $Ba^{2+}$, $Mg^{2+}$, $Sr^{2+}$, $Zn^{2+}$ oder $Al^{3+}$ oder $N^+H(R_1)(R_2)(R_3)$, worin $R_1$, $R_2$ und $R_3$ Wasserstoff, $C_1$-$C_{22}$-Alkyl oder $C_5$-$C_6$-Cycloalkyl sind, bedeuten und R bei m = Null Wasserstoff, Methyl, tert.-Butyl oder Methoxy und bei m = 1 Wasserstoff ist.

Bevorzugt sind erfindungsgemässe Mischkristalle aus mindestens zwei voneinander verschiedenen Verbindungen der Formel

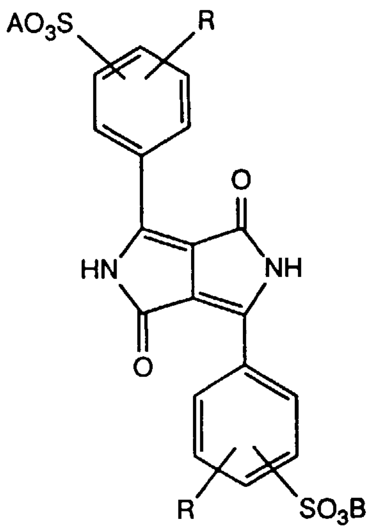

(VI),

worin R Wasserstoff oder Methyl ist.

A und B haben dabei die in der oben genannten Bevorzugung angegebene Bedeutung.

Ebenfalls bevorzugt sind erfindungsgemässe Mischkristalle aus mindestens zwei voneinander verschiedenen Verbindungen der Formel II, worin A und B die in der oben genannten Bevorzugung angegebene Bedeutung haben.

Besonders bevorzugt sind erfindungsgemässe Mischkristalle aus mindestens zwei voneinander verschiedenen Verbindungen der Formel

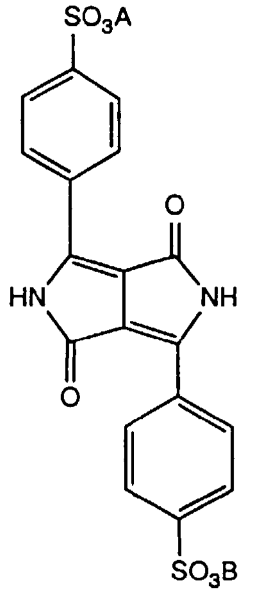

(VII).

A und B haben dabei die in der oben genannten Bevorzugung angegebene Bedeutung, sie bedeuten aber ganz besonders bevorzugt $\frac{Sr^{2+}}{2}$ und $N^+H_3C_8H_{17}$ bei einer Zusammensetzung

$$\left(\frac{Sr^{2+}}{2}\right)_{1-x}\left(N^+H_3C_8H_{17}\right)_x ,$$

worin x eine Zahl zwischen 0,4 und 0,6 bedeutet.

Mischkristalle werden durch ihr Röntgenbeugungsdiagramm gekennzeichnet, welches sich sowohl von demjenigen der Einzelkomponenten des Mischkristalls als auch von demjenigen ihres physikalischen Gemisches unterscheidet.

Das Röntgenbeugungsdiagramm der erfindungsgemässen Mischkristalle ist durch andere Linien gekennzeichnet als diejenigen, welche die Röntgenbeugungsdiagramme des entsprechenden physikalischen Gemischs und der entsprechenden Einzelkomponenten charakterisieren.

Die erfindungsgemässen Mischkristalle können als Pigmente zum Färben von hochmolekularem organischem Material verwendet werden. Dabei lassen sie sich im allgemeinen direkt in der Form einsetzen, wie sie nach ihrer Synthese anfallen. Je nach Verwendungszweck kann man die erfindungsgemässen Mischkristalle in eine dekkendere oder transparentere Form überführen.

Hochmolekulare organische Materialien, die mit den erfindungsgemässen Mischkristallen gefärbt bzw. pigmentiert werden können, sind z.B. Celluloseether und -ester, wie Ethylcellulose, Nitrocellulose, Celluloseacetat, Cellulosebutyrat, natürliche Harze oder Kunstharze, wie Polymerisationsharze oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, ABS, Polyphenylenoxide, Gummi, Casein, Silikon und Silikonharze, einzeln oder in Mischungen.

Besonders geeignet sind die erfindungsgemässen Mischkristalle zum Einfärben von Polyvinylchlorid und Polyolefinen, wie Polyethylen und Polypropylen sowie von ABS.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen Mischkristalle als Toner oder in Form von Präparaten einzusetzen.

Bezogen auf das zu pigmentierende hochmolekulare organische Material kann man die erfindungsgemässen Mischkristalle in einer Menge von 0,01 bis 30 Gew.%, vorzugsweise von 0,1 bis 10 Gew.%, einsetzen.

Die Pigmentierung der hochmolekularen organischen Substanzen mit den erfindungsgemässen Mischkristallen erfolgt beispielsweise derart, dass man solche Mischkristalle gegebenenfalls in Form von Masterbatches diesen Substraten unter Verwendung von Walzwerken, Misch-oder Mahlapparaten zumischt. Das pigmentierte Material wird

hierauf nach an sich bekannten Verfahren, wie Kalandrieren, Pressen, Strangpressen, Streichen, Giessen oder Spritzgiessen, in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können vor oder nach der Einverleibung der erfindungsgemässen Mischkristalle in die Polymeren eingearbeitet werden. Zwecks Erzielung verschiedener Farbtöne ist es ferner möglich, den hochmolekularen organischen Stoffen neben den erfindungsgemässen Mischkristallen noch Füllstoffe bzw. andere farbgebende Bestandteile, wie Weiss-, Bunt- oder Schwarzpigmente, in beliebigen Mengen zuzufügen.

Zum Pigmentieren von Lacken, Anstrichstoffen und Druckfarben werden die hochmolekularen organischen Materialien und die erfindungsgemässen Mischkristalle gegebenenfalls zusammen mit Zusatzstoffen, wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

In Färbungen, beispielsweise von Polyvinylchlorid oder Polyolefinen, zeichnen sich die erfindungsgemässen Mischkristalle durch gute allgemeine Pigmenteigenschaften, wie hohe Farbstärke und Reinheit, gute Migrations- und Wetterbeständigkeit sowie gute Deckkraft, aber insbesondere durch ausserordentliche Dispergierbarkeit, Hitze- und Lichtbeständigkeit aus.

Die nachfolgenden Beispiele erläutern die Erfindung.

Beispiel 1: 157 g wässrige Paste, etwa 31,1 %ig, aus dem Diketopyrrolopyrroldisulfonsäuredinatriumsalz der Formel

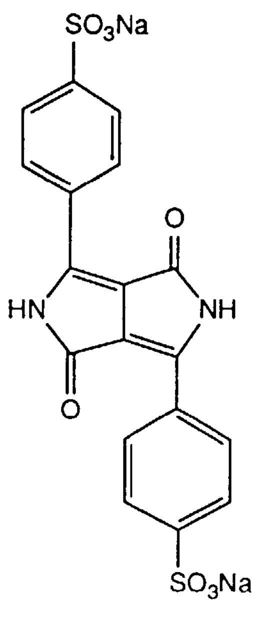

(VIII)

werden in 1900 ml entionisiertem Wasser 25 Minuten bei Raumtemperatur verrührt. Die rote Suspension wird mit 100 ml 1N Salzsäure versetzt und 10 Minuten weiter gerührt. Man gibt 27 g fein pulverisiertes Stearylamin zu und erhitzt auf 80°C. Es findet eine schwache Aufhellung statt. Die rote Suspension wird 2 Stunden bei 80°C gerührt und heiss durch einen Stoff-Filter filtriert. Das Nutschgut wird mit 4000 ml entionisiertem Wasser gewaschen und im Vakuum bei 80°C getrocknet. Man erhält 74 g (100 % der Theorie) eines roten, weichen Pulvers das folgende Analysenwerte ergibt:

Die berechneten Werte beziehen sich auf eine Zusammensetzung $Na^+$ zu $N^+H_3C_{18}H_{37}$ im Molverhältnis 1:1 (d.h. x = 0,5).

| Analyse: | C | H | N | S | Na |
|---|---|---|---|---|---|
| Berechnet: | 58,44 % | 6,81 % | 5,68 % | 8,67 % | 3,11 % |
| Gefunden: | 58,50 % | 7,10 % | 5,70 % | 8,24 % | 2,36 % |

Das Röntgenbeugungsdiagramm wurde auf einem Röntgendifraktometer D500 der Firma Siemens mit Cu-K-alpha-Strahlung aufgenommen. In der nachfolgenden Tabelle sind die d-Werte der stärksten Linien (d > 3,0 Å) angegeben, zusammen mit den von Auge geschätzten relativen Linienintensitäten.

| d-Wert in Å | Intensität |
|---|---|
| 19,2 | mittel |
| 12,8 | schwach |
| 9,6 | stark |
| 8.5 | schwach |
| 7.9 | schwach |
| 7.3 | mittel |
| 6,4 | schwach |
| 5,2 | schwach |
| 5,0 | sehr stark |
| 4,6 | schwach |
| 4,3 | mittel |
| 4,1 | schwach |
| 4,0 | schwach |
| 3,7 | mittel |
| 3,6 | mittel |
| 3,4 | sehr stark |
| 3,2 | schwach |
| 3,1 | schwach |
| 3,0 | schwach |

Beispiel 2:

Beispiel 1 wird wiederholt mit der einzigen Ausnahme, dass anstelle des Stearylamins unter sonst gleichen Bedingungen 11 ml Cyclohexylamin verwendet werden.

Man erhält 53 g (90 % d.Th.) eines roten Pulvers, enthaltend 1 Aequivalent Kristallwasser, mit folgenden Analysenwerte

| Analyse: | C | H | N | S | Na |
|---|---|---|---|---|---|
| Berechnet: | 49,06 % | 4,46 % | 7,15 % | 10,91 % | 3,91 % |
| Gefunden: | 49,00 % | 4,27 % | 7,07 % | 10,95 % | 4,62 % |

Die berechneten Werte beziehen sich auf eine Zusammensetzung $Na^+$ zu $N^+H_3C_6H_{11}$ im Molverhältnis 1:1.

Beispiel 3-6: *a* wird in einem Gemisch von 3000 ml Wasser und 150 ml n-Butanol bei Raumtemperatur suspendiert und auf 90°C erhitzt. Nach einer Stunde wird *b* zugegeben und das Gemisch nochmals eine Stunde bei 90°C gerührt. Nun wird ein Gemisch von c und *d* zugegeben und die etwas viskose, aber immer noch gut rührbare rote Suspension, wird weitere 18 Stunden bei 90°C gehalten. Das Produkt wird über ein Hartpapierfilter filtriert, mit 2000 ml Wasser gewaschen und im Vakuum bei 120°C getrocknet.

*a*, *b*, *c* und *d* entsprechen in den Beispielen 3-6 jeweils folgenden Angaben:

Beispiel 3:

*a*: 122 g der Verbindung der Formel VIII (Beispiel 1), Gehalt: 93 %.

*b*: 253 ml 1 N HCl.

*c*: 34 g $SrCl_2 \cdot 6H_2O$.

*d:* 39 ml n-Octylamin.

Man erhält 137 g (93 % d.Th.) dunkelrotes Pulver, enthaltend 1,25 Aequivalente Kristallwasser, mit folgenden Analysenwerten:

| Analyse: | C | H | N | S | Sr |
|---|---|---|---|---|---|
| Berechnet: | 48,57 % | 5,09 % | 6,53 % | 9,97 % | 6,81 % |
| Gefunden: | 48,38 % | 4,64 % | 6,41 % | 10,20 % | 7,69 % |

½ $Sr^{2+}$: $N^+H_3C_8H_{19}$ = 1:1.

Beispiel 4:

*a*: 122 g der Verbindung der Formel VIII (Beispiel 1), Gehalt: 93 %.
*b*: 253 ml 1 N HCl.
*c*: 25 g $CaCl_2 \cdot 6H_2O$.
*d*: 63 g Stearylamin, fein gepulvert.

Man erhält 166 g (96 % d.Th.) rotes Pulver, enthaltend 1 Aequivalent Kristallwasser, mit folgenden Analysenwerten:

| Analyse: | C | H | N | S | Ca |
|---|---|---|---|---|---|
| Berechnet: | 57,27 % | 6,94 % | 5,56 % | 8,49 % | 2,65 % |
| Gefunden: | 57,20 % | 6,70 % | 5,80 % | 8,70 % | 2,56 % |

½ $Ca^{2+}$: $N^{+}H_3C_{18}H_{37}$ = 1:1.

Beispiel 5:

*a*: 122 g der Verbindung der Formel VIII (Beispiel 1), Gehalt: 93 %.
*b*: 253 ml 1 N HCl.
*c*: 28 g $MgCl_2 \cdot 6H_2O$.
*d*: 39 g n-Octylamin

Man erhält 122 g (88 % d.Th.) hellrotes Pulver, enthaltend 1 Aequivalent Kristallwasser, mit folgenden Analysenwerten:

| Analyse: | C | H | N | S | Mg |
|---|---|---|---|---|---|
| Berechnet: | 51,46 % | 5,32 % | 6,92 % | 10,57 % | 2,00 % |
| Gefunden: | 51,50 % | 5,40 % | 6,96 % | 10,24 % | 1,58 % |

½ $Mg^{2+}$: $N^{+}H_3C_8H_{19}$ = 1:1.

Beispiel 6:

*a*: 121 g der Verbindung der Formel

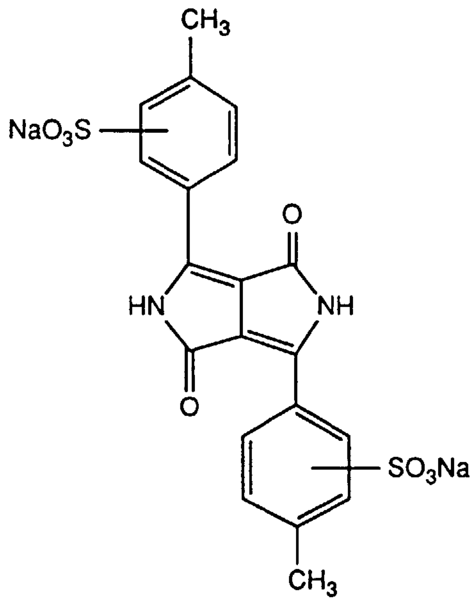

, Gehalt:99 %.

Gehalt:99 %.
*b*: 266 ml 1 N HCl.
*c*: 31 g $SrCl_2 \cdot 6H_2O$.
*d*: 68 g Stearylamin, fein gepulvert.

Man erhält 196 g (97 % d.Th.) eines dunkelroten Pulvers, mit folgenden Analysenwerten:

| Analyse: | C | H | N | S | Sr |
|---|---|---|---|---|---|
| Berechnet: | 57,86 % | 6,90 % | 5,33 % | 8,13 % | 5,55 % |
| Gefunden: | 57,98 % | 7,72 % | 5,22 % | 7,24 % | 4,00 % |

½ $Sr^{2+}$: $N^+H_3C_{18}H_{37}$ = 1:1.

Alle in den Beispielen 1-6 erhaltenen Produkte können als Mischkristalle bezeichnet werden, da deren Röntgenbeugungsdiagramme sich stets auf signifikante Art und Weise von denjenigen der entsprechenden Gemische aus den Einzelkomponenten, d.h. den reinen Metall- und Alkylammoniumsalzen, unterscheiden.

Alle in den Beispielen 1-6 erwähnten Produkte zeigen in Polyvinylchlorid, Polyolefinen und ABS sehr gute Migrations-, Hitze- und Lichtbeständigkeiten.

## Patentansprüche

1. Mischkristalle aus mindestens zwei voneinander verschiedenen Verbindungen der Formel

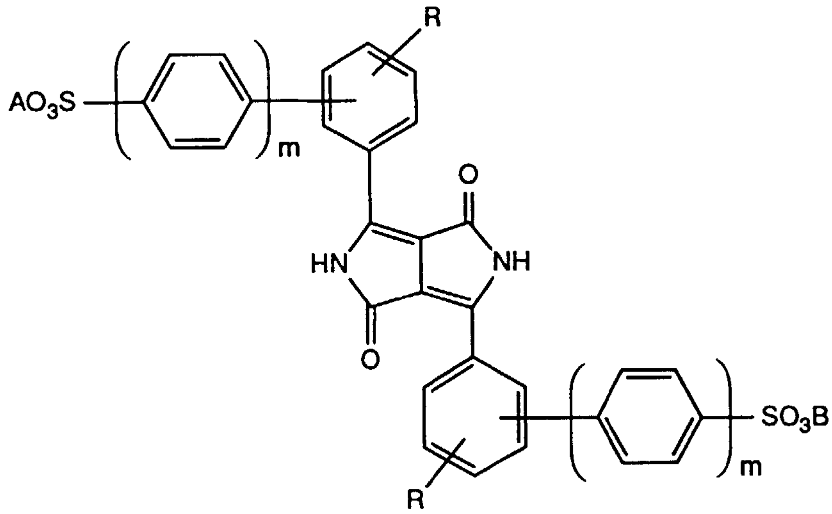

(I),

worin A und B unabhängig voneinander ein Kation der Formeln

$$\frac{M^{+n}}{n} \quad \text{oder} \quad N^{+}H(R_1)(R_2)(R_3)$$

darstellen, M ein ein-, zwei- oder dreiwertiges Metallkation, n die Zahlen 1, 2 oder 3, $R_1$, $R_2$ und $R_3$ unabhängig voneinander Wasserstoff, $C_1$-$C_{22}$-Alkyl, $C_7$-$C_{24}$-Aralkyl, $C_5$-$C_6$-Cycloalkyl oder $C_6$-$C_{18}$-Aryl bedeuten, m Null oder 1 ist und R Wasserstoff, $C_1$-$C_4$-Alkyl oder $C_1$-$C_4$-Alkoxy bedeutet, dadurch gekennzeichnet, dass in den die Mischkristalle bildenden Verbindungen der Formel I die Kationen A und B eine Zusammensetzung

$$\left(\frac{M^{+n}}{n}\right)_{1-x}\left[N^{+}H(R_1)(R_2)(R_3)\right]_x$$

aufweisen, worin x eine Zahl zwischen 0,2 und 0,8, bedeutet und dass sich das Röntgenbeugungsdiagramm der Mischkristalle vom Röntgenbeugungsdiagramm der Gemische der entsprechenden Verbindungen in welchen x 1 beziehungsweise Null bedeutet, unterscheidet.

**2.** Mischkristalle gemäss Anspruch 1, dadurch gekennzeichnet, dass X eine Zahl zwischen 0,35 und 0,65 ist.

**3.** Mischkristalle gemäss Anspruch 2 aus mindestens zwei voneinander verschiedenen Verbindungen der Formel I, worin A und B unabhängig voneinander ein Kation ausgewählt aus $Na^+$, $Ca^{2+}$, $Ba^{2+}$, $Mg^{2+}$, $Sr^{2+}$, $Zn^{2+}$ oder $Al^{3+}$ oder $N^+H(R_1)(R_2)(R_3)$, worin $R_1$, $R_2$ und $R_3$ Wasserstoff, $C_1$-$C_{22}$-Alkyl oder $C_5$-$C_6$-Cycloalkyl sind, bedeuten und R bei m = Null Wasserstoff, Methyl, tert.-Butyl oder Methoxy und bei m = 1 Wasserstoff ist.

**4.** Mischkristalle gemäss Anspruch 3 aus mindestens zwei voneinander verschiedenen Verbindungen der Formel

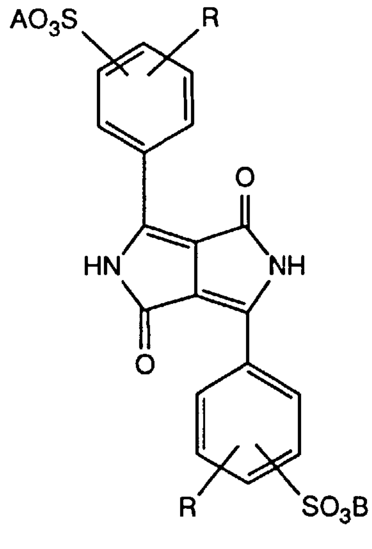

(VI),

worin R Wasserstoff oder Methyl ist.

**5.** Mischkristalle gemäss Anspruch 3 aus mindestens zwei voneinander verschiedenen Verbindungen der Formel

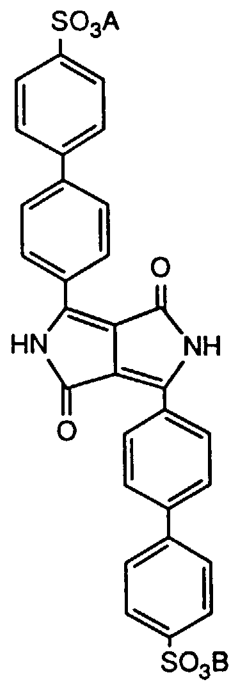

(II)

**6.** Mischkristalle gemäss Anspruch 4 aus mindestens zwei voneinander verschiedenen Verbindungen der Formel

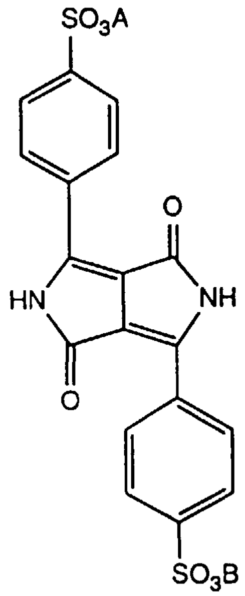

(VII)

7. Mischkristalle gemäss Anspruch 6, dadurch gekennzeichnet, dass A und B unabhängig voneinander $\frac{Sr^{2+}}{2}$ und $N^{+}H_3C_8H_{17}$ bedeuten, bei einer Zusammensetzung

$$\left(\frac{Sr^{2+}}{2}\right)_{1-x}\left(N^{+}H_3C_8H_{17}\right)_x ,$$

worin x eine Zahl zwischen 0,4 und 0,6 bedeutet.

8. Mit Mischkristallen gemäss Anspruch 1 pigmentiertes hochmolekulares organisches Material.

9. Hochmolekulares organisches Material gemäss Anspruch 8, dadurch gekennzeichnet, dass es sich um Polyolefine oder ABS handelt.

**Claims**

1. Mixed crystals of at least two different compounds of formula

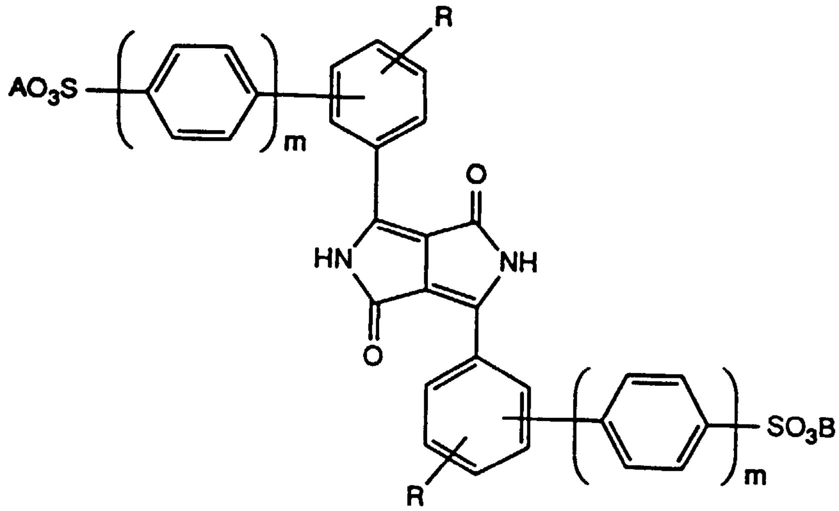

(I),

wherein A and B are each independently of the other a cation formula

$$\frac{M^{+n}}{n} \quad \text{or} \quad N^{+}H(R_1)(R_2)(R_3),$$

M is a mono-, di- or trivalent metal cation, n is 1, 2 or 3, $R_1$, $R_2$ and $R_3$ are each independently of one another hydrogen, $C_1$-$C_{22}$alkyl, $C_7$-$C_{24}$aralkyl, $C_5$-$C_6$cycloalkyl or $C_6$-$C_{18}$aryl, m is zero or 1, and R is hydrogen, $C_1$-$C_4$alkyl or $C_1$-$C_4$alkoxy, wherein, in the compounds of the formula I that form the mixed crystals, the cations A and B are of a composition

$$\left(\frac{M^{+n}}{n}\right)_{l-x}\left[N^{+}H(R_1)(R_2(R_3)\right]_x$$

in which x is a number between 0.2 and 0.8 and wherein the X-ray diffraction pattern of the mixed crystals differs from that of the mixtures of corresponding compounds in which x is 1 or zero.

**2.** Mixed crystals according to claim l, wherein x is a number between 0.35 and 0.65.

**3.** Mixed crystals according to claim 2 of at least two different compounds of formula I wherein A and B are each independently of the other selected from $Na^{+}$, $Ca^{2+}$, $Ba^{2+}$, $Mg^{2+}$, $Sr^{2+}$, $Zn^{2+}$ or $Al^{3+}$ or $N^{+}H(R_1)(R_2)(R_3)$ in which $R_1$, $R_2$ and $R_3$ are hydrogen, $C_1$-$C_{22}$alkyl or $C_5$-$C_6$cycloalkyl, and R, when m = zero, is hydrogen, methyl, tert-butyl or methoxy and, when m = 1, is hydrogen.

**4.** Mixed crystals according to claim 3 of at least two different compounds of formula

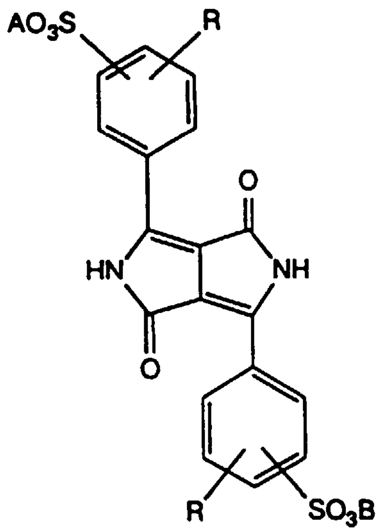

(VI),

wherein R is hydrogen or methyl.

5. Mixed crystals according to claim of at least two different compounds of formula

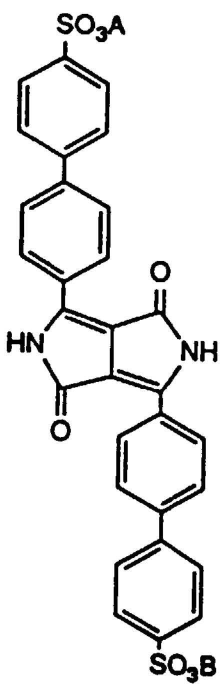

(II)

6. Mixed crystals according to claim 4 of at lest two different compounds of formula

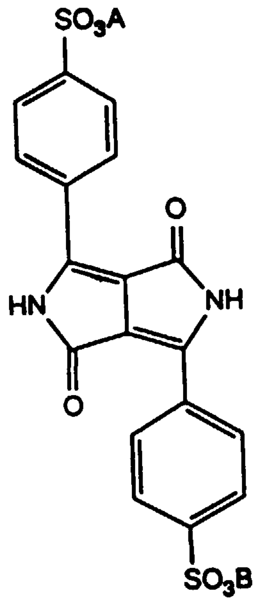

(VII)

**7.** Mixed crystals according to claim 6, wherein A and B are each independently of the other

$$\left(\frac{Sr^{2+}}{2}\right)$$

and $N^+H_3C_8H_{17}$ in the case of a composition

$$\left(\frac{Sr^{2+}}{2}\right)_{1-x}\left(N^+H_3C_8H_{17}\right)_x$$

in which x is a number between 0.4 and 0.6.

**8.** Organic material of high molecular weight which is pigmented with mixed crystals according to claim 1.

**9.** Organic material of high molecular weight according to claim 8 which is a polyolefin or ABS.

**Revendications**

**1.** Cristaux mixtes d'au moins deux composés différents l'un de l'autre de formule

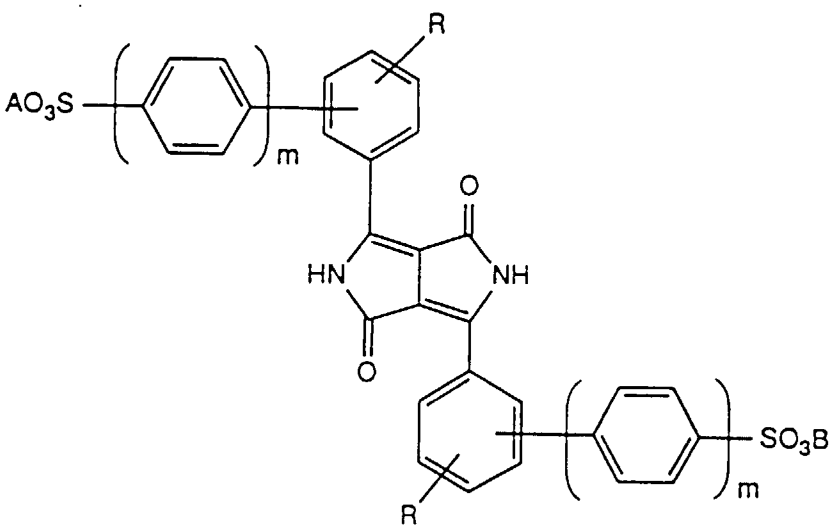

(I),

dans laquelle A et B représentent indépendamment l'un de l'autre un cation de formules

$$\frac{M^{+n}}{n} \quad \text{ou} \quad N^{+}H(R_1)(R_2)(R_3),$$

M un cation métallique mono, bi ou trivalent, n les nombres 1, 2 ou 3, $R_1$, $R_2$ et $R_3$ représentent indépendamment les uns des autres un atome d'hydrogène, un groupe alkyle en $C_1$-$C_{22}$, aralkyle en $C_7$-$C_{24}$, cycloalkyle en $C_5$-$C_6$ ou aryle en $C_6$-$C_{18}$, m est zéro ou 1 et R représente un atome d'hydrogène, un groupe alkyle en $C_1$-$C_4$ ou alcoxy en $C_1$-$C_4$, caractérisés en ce que, dans les composés de formule I constituant les cristaux mixtes, les cations A et B présentent une combinaison

$$\left(\frac{M^{+n}}{n}\right)_{1-x}\left[N^{+}H(R_1)(R_2)(R_3)\right]_x,$$

dans laquelle x représente un nombre entre 0,2 et 0,8, et en ce que le diagramme de diffraction des rayons X des cristaux mixtes se distingue du diagramme de diffraction des rayons X des mélanges correspondants dans lesquels x représente 1 ou zéro.

**2.** Cristaux mixtes selon la revendication 1, caractérisés en ce que x est un nombre entre 0,35 et 0,65.

**3.** Cristaux mixtes selon la revendication 2 constitué d'au moins deux composés différents de formule I, dans laquelle A et B représentent un cation choisi parmi $Na^+$, $Ca^{2+}$, $Ba^{2+}$, $Mg^{2+}$, $Sr^{2+}$, $Mn^{2+}$, $Zn^{2+}$ ou $Al^{3+}$ ou $N^+H(R_1)(R_2)(R_3)$, dans laquelle $R_1$, $R_2$ et $R_3$ représentent un atome d'hydrogène, un groupe alkyle en $C_1$-$C_{22}$ ou cycloalkyle en $C_5$-$C_6$ et R représente un atome d'hydrogène, un groupe méthyle, tert.-butyle ou méthoxy pour m = zéro et est un atome d'hydrogène pour m = 1.

**4.** Cristaux mixtes selon la revendication 3, constitués d'au moins deux composés différents entre eux de formule

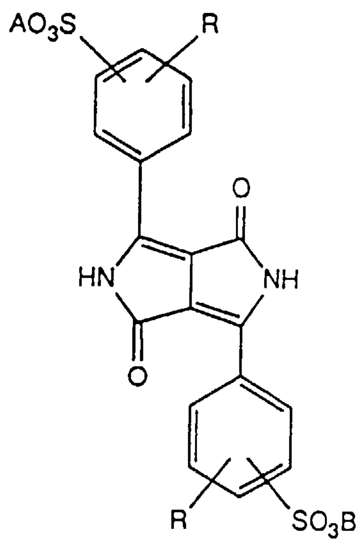

(VI),

dans laquelle R est un atome d'hydrogène ou un groupe méthyle.

5. Cristaux mixtes selon la revendication 3 constitués d'au moins deux composés différents entre eux de formule

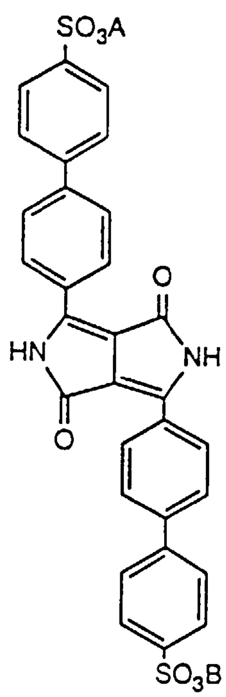

(II)

6. Cristaux mixtes selon la revendication 4 constitués d'au moins deux composés différents entre eux de formule

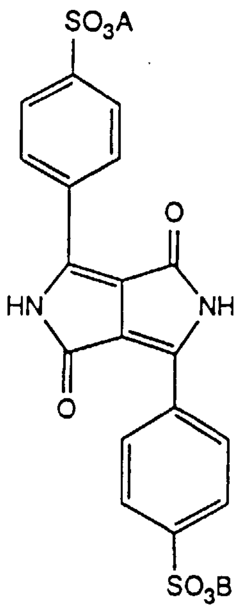

(VII)

**7.** Cristaux mixtes selon la revendication 6, caractérisés en ce que A et B représentent indépendamment l'un de l'autre $\frac{Sr^{2+}}{2}$ et $N^+H_3C_8H_{17}$, dans une combinaison

$$\left(\frac{Sr^{2+}}{2}\right)_{1-x}\left[N^+H_3C_8H_{17}\right]_x ,$$

dans laquelle x représente un chiffre entre 0,4 et 0,6.

**8.** Matériau organique de molécularité élevée pigmenté avec des cristaux mixtes selon la revendication 1.

**9.** Matériau organique de molécularité élevée selon la revendication 8, caractérisé en ce qu'il s'agit de polyoléfines ou d'ABS.